(19) Europäisches Patentamt
European Patent Office
Office européen des brevets

(11) **EP 4 309 514 A1**

(12) **EUROPEAN PATENT APPLICATION**
published in accordance with Art. 153(4) EPC

(43) Date of publication:
**24.01.2024 Bulletin 2024/04**

(21) Application number: **22770671.0**

(22) Date of filing: **15.03.2022**

(51) International Patent Classification (IPC):
**A23L 33/00** (2016.01) **A61K 31/20** (2006.01)
**A61K 31/201** (2006.01) **A61K 31/202** (2006.01)

(52) Cooperative Patent Classification (CPC):
**A23L 33/00; A61K 31/20; A61K 31/201; A61K 31/202**

(86) International application number:
**PCT/ES2022/070146**

(87) International publication number:
**WO 2022/195145 (22.09.2022 Gazette 2022/38)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **17.03.2021 ES 202130236**
**30.11.2021 ES 202131111**

(71) Applicant: **Cárnicas Joselito, S.A.**
**37770 Guijuelo Salamanca (ES)**

(72) Inventors:
• **MIGUEL CASTRO, Marta**
**37770 Guijuelo (Salamanca) (ES)**

• **BRUNA GARCÍA, Eva**
**37770 Guijuelo (Salamanca) (ES)**
• **ISABEL REDONDO, Beatriz**
**37770 Guijuelo (Salamanca) (ES)**
• **GÓMEZ MARTÍN, José**
**37770 Guijuelo (Salamanca) (ES)**
• **GÓMEZ SÁNCHEZ, José**
**37770 Guijuelo (Salamanca) (ES)**
• **SABATER MUÑOZ, Beatriz**
**37770 Guijuelo (Salamanca) (ES)**

(74) Representative: **Ungria López, Javier**
**Avda. Ramón y Cajal, 78**
**28043 Madrid (ES)**

(54) **PORK FAT EXTRACT, METHOD OF OBTAINMENT, COMPOSITIONS COMPRISING SAME AND USES THEREOF**

(57) The invention relates to an extract from pork ham fat, preferably from fat trimmings, which is a by-product of slicing ham, and more preferably from ham from Iberian pigs, at best fed on grass and acorns. This extract has very advantageous culinary properties, is very healthy and may have a monounsaturated fatty acid content, such as oleic acid, of between 60%-62%. The invention also relates to a method for obtaining the extract, its use as a food or culinary ingredient, and the food composition comprising it, such as a meat product, a dairy product, a dairy-derived product or a sauce, inter alia. Thanks to its properties, the extract can act or be contained in a functional or nutraceutical food. The invention also relates to a pharmaceutical composition comprising the extract in a specific amount and its use in medicine.

Fig. 1

## Description

### Technical field

The invention falls within the scope of the food industry, more specifically in the meat industry and more particularly in the pork industry. It deals specifically with formulations used in the field of diet based on pork fat, and it is specifically an extract (mainly, food extract) from said pork fat, which can be used as a food composition of the seasoning and/or flavouring type. The fat can be from any pig, although more preferably it is an Iberian pig (*sus scrofa mediterranea*), preferably fed on acorns and pastures (grass), a fattening known as the "montanera" period, said fattening time possibly being 36 months in the best of the embodiments. The fat is specifically extracted from cured pork ham. Given the composition of this extract, it also has nutraceutical properties, as it acts as a functional food of natural origin that improves health and prevents certain diseases.

The invention also falls within the scope of the pharmaceutical industry. It specifically deals with formulations with medical use that come from the food industry, and are composed exclusively of natural ingredients, based on pork fat. The fat can be from any pig, although, as said, more preferably it is an Iberian pig (*sus scrofa mediterranea*), preferably fed on acorns and pastures (grass), a fattening known as the "montanera" period, said fattening time possibly being 36 months in the best of the embodiments. The fat is obtained specifically from cured pork ham. Given the composition of this extract of natural origin, it has been proven to have pharmaceutical properties with benefits to the body derived from its antioxidant capacity.

### Background

According to data from the National Meat Industry Association in Spain (ANICE) and the Ministry of Agriculture, Fishing and Food (MAPAMA), pork production is the largest Spanish meat activity, which reached a figure of 4,521,588 tons in 2018 (ANICE and MAPAMA, 2019). In the context of the national pork sector, the subsector dedicated to production, transformation and preparation of products derived from the Iberian pig represents an especially relevant group in Spain, not only from an economic point of view, but also because it is a cultural and gastronomic legacy of incalculable value. Today, products derived from the Iberian pig are the most representative traditional Spanish meat products, and are rapidly spreading to the most prestigious "gourmet" markets (Ventanas, S., Ventanas, J., Ruiz, J. and Estévez, M. (2005). Iberian pigs for the development of high-quality cured products. Res. Devel. Agricultural & Food Chem., 6, 1-27).

One of the most important and prestigious producers of Iberian meat derivatives worldwide is the company Cárnicas Joselito S.L. This company, founded in Salamanca in 1868, has more than 170,000 hectares of Dehesa grazing with suitable weather conditions for the production of high-quality Iberian meat products (hams, shoulder, chorizos, dried sausage or cured pork loin, inter alia). The Joselito® pig belongs to the Iberian breed, which is a pig breed native to the Iberian Peninsula raised mainly in the southwest of the Peninsula, and in particular, Joselito has developed its own selection program, all of its breeders are registered in the herd book of the Spanish Association of Iberian Pig Breeders (AECERIBER). These animals traditionally feed on acorns (in Spain, *Quercus ilex, Quercus rotundifolia* and *Quercus suberosus*) and pastures in a broad production system, which causes the accumulation of high levels of oleic acid and tocopherols in their tissues and the production of excellent quality meat.

In order to produce meat products, in addition to the importance of breed and diet, the manufacturing process that, over the years, has become a mix of tradition and cutting-edge is also important. The fattening of animals in the dehesa grazing mainly takes place with acorns and grass, which is a process known as the "montanera" period, and that is decisive in the composition, nutritional and sensory quality of Iberian products (Jiménez-Colmenero, F., Ventanas, J. and Toldrá, F. (2010). Nutritional composition of dry-cured ham and its role in a healthy diet. Meat Science, 84, 585-593).

The diet of the pig during the montanera period and the curing time of the ham, which can be more than 36 months, provide this product with unique nutritional and organoleptic characteristics. Both factors are determining factors in the composition of fatty acids and also provide a significant presence of natural antioxidants in the ham, such as vitamin E and its isomers, which can be beneficial not only for the animal's body, but also for the health of those who consume it. It has been described that acorn-fed ham (which is the name given to ham from montanera pigs) has a high content of monounsaturated fatty acids (MUFA) and polyunsaturated fatty acids (PUFA), mainly highlighting the levels of oleic acid and linoleic acid (Ventanas et. al., 2005). In this context, several scientific studies have shown that fats with high MUFA content produce a beneficial effect on blood cholesterol, by causing an increase in HDL cholesterol (high density lipoproteins) and a reduction in LDL cholesterol (low density lipoproteins). Therefore, it contributes to reducing the risk of various cardiovascular diseases (Rodríguez-González, M., Tárraga, M.L., Madrona, F., Sadek, I.M., Celada, C., and Tárraga, P.J. (2019). Effects of the Mediterranean diet on the cardiovascular risk factors. JONNPR. 4(1), 25-51). It should also be noted that, in products derived from the Iberian pig, the concentrations of saturated fatty acids, the intake of which is related to an increase in cardiovascular risk, are lower than those presented by hams from white pigs. Regarding the antioxidant compounds present in ham, they act as protective agents against excess reactive oxygen species (ROS) and are capable of neutralising free radicals, inhibit enzymes involved in the formation of ROS such as xanthine oxidase, lipoxygenase and NADPH oxidase, can prevent cell death or increase the production of endogenous antioxidants (Jiménez-Colmenero, F. et al., 2010). In previous studies carried out with Iberian ham, specifically from the Joselito® brand, it has been shown that its consumption produces a reduction in average blood pressure and triglycerides. Furthermore, the intake of Joselito® ham has been related to an improvement in oxidative stress, due to the presence of specific antioxidant substances such as the aforementioned Vitamin E and the high content of oleic acid, that help prevent lipid oxidation (Mayoral, P., Martinez-Salgado, C.S., Santiago, J.M., Rodríguez-Hernández, M.V., García-Gómez, M.L., Morales, A., López-Novoa, J.M., and Marcías-Núñez, J.F. (2003). Effect of ham protein substitution on oxidative stress in older adults. Nutr Health Aging, 7(2), 84-9).

Iberian acorn-fed ham, such as Joselito® ham, in addition to being a product with high organoleptic and nutritional characteristics, exhibits a high efficiency, since the entire ham can be used, some of them for direct consumption and others to improve the sensory and/or nutritional quality of prepared dishes. Among the parts that are not intended for direct consumption are the fat trimmings of the ham. This fat is considered a by-product that comes from slicing the ham, which is traditionally used to prepare broths and other spoon dishes typical of Spanish gastronomy, such as legume stews or chacinero rice. These fat trimmings, being a by-product of slicing ham, are also rich in MUFA and antioxidants, and maintain the sensory qualities of Iberian ham, such that its reuse and revaluation, beyond the uses currently given to it, could be interesting.

To date, there are few studies carried out that use ham from Iberian pigs fed with acorns as the subject of study, and most of the studies have been carried out on cured ham. The studies carried out to date on hams from Iberian pigs fed with acorns have mainly focused on evaluating how acorn intake affects the increase in the amount and proportion of monounsaturated fatty acids in ham (Ventanas, S., Ventanas, J., Tovar, J., Garcia, C. and Estévez, M. (2007). Extensive feeding versus oleic acid and tocopherol enriched mixed diets for the production of Iberian dry-cured hams: Effect on chemical composition, oxidative status and sensory traits. Meat Science, 77, 246-256) and researching the relationship between acorn consumption and the aroma profile of ham, and only one clinical study has been found in which the intake of ham from pigs fed or not fed with acorns on vascular health is compared (Saban-Ruiz, J., Fabregate-Fuente, M., Fabregate-Fuente, R. Andrés-Castillo, A., Palomino, A., Barrio-Carreras, D., Martín-Fernández, L. Alramirano, F., Férnandez-Férnandez, C. and Andrés-Lacueva, C. (2017). Iberian cured-ham consumption improves endothelial function in healthy subjects. J Nutr Health Aging 21, 1277-1283). However, there are no studies on hams from Iberian pigs fed with acorns in which the revaluation or use of any of the waste or by-products generated after its production is evaluated or proposed both from a nutritional and health point of view, as well as from a sensory and gastronomic point of view. The company Cárnicas Joselito S.L. has been focusing on innovation and tradition in its products for decades, and today it is the main reference in research on the Iberian pig and all the potential uses of this product. For this reason, given the deficiencies detected in this field of research, the present invention has been developed in order to offer a quality raw material for food, which is an extract that can be used as a food ingredient or food supplement of great nutritional and sensory value. Furthermore, it can be used as a nutraceutical food as it is a functional food with beneficial properties for health. Said product is based on the use of fat from cured pork ham, preferably Iberian, and more specifically from Joselito® Iberian pork fat; and specifically being the fat trimmings, for the preparation of a new functional ingredient with a high sensory quality, rich in antioxidant compounds and healthy fats, which allows this by-product of the food industry to be revalued and, furthermore, contributes to the improvement of health, well-being and quality of life of consumers.

Most organisms are exposed to oxidative stress, which is caused by the generation of reactive oxygen species (ROS) after respiration or after exposure to UV light or other ionizing radiation. These are partially reduced forms of molecular oxygen, highly reactive and with great capacity to exert cellular damage. Its production may be a consequence of the cells' own metabolic processes (mainly the metabolism of the mitochondria), or formed by exposure to certain environmental factors such as UV light, pollution, tobacco and other environmental pollutants such as exposure to heavy metals (Martorell, P., Forment, J.V., de Llanos, R., Montón, F., Llopis, S., González, N., Genovés, S., Cienfuegos, E., Monzó, H., and Ramón, D. (2011). Use of Saccharomyces cerevisiae and Caenorhabditis elegans as Model Organisms to Study the Effect of Cocoa Polyphenols in the Resistance to Oxidative Stress. J. Agric. Food Chem., 59(5), 2077-2085; Takagi, H., and Kitagaki, H. (Eds.). (2015). Stress Biology of Yeasts and Fungi. Tokyo, Japan). The excess of these oxidative species is quickly counteracted through antioxidant defence mechanisms, giving rise to an oxidation-reduction balance essential for living organisms, known as *redox balance*. These antioxidant systems can be of endogenous origin, the following being of note: superoxide dismutase, catalase, glutathione peroxidase, peroxiredoxins and non-enzymatic antioxidants such as coenzyme Q10, melatonin, albumin, bilirubin and uric acid (Martorell, P., Forment, J.V., de Llanos, R., Montón, F., Llopis, S., González, N., Genovés, S., Cienfuegos, E., Monzó, H., and Ramón, D. (2011). Use of Saccharomyces cerevisiae and Caenorhabditis elegans as Model Organisms to Study the Effect of Cocoa Polyphenols in the Resistance to Oxidative Stress. J. Agric. Food Chem., 59(5), 2077-2085; Carocho, M., and Ferreira, I.C.F.R (2013). A review on antioxidants, prooxidants and related controversy: Natural and synthetic compounds, screening and analysis methodologies and future perspectives. Food and Chemical Toxicology 51, 5-25; Takagi, H., and Kitagaki, H. (Eds.).

(2015). Stress Biology of Yeasts and Fungi. Tokyo, Japan). Moreover, there are also exogenous antioxidants that can be obtained through diet and nutritional supplements. In this group, the following stand out: carotenoids, flavonoids, phenolic compounds, organosulphur compounds and vitamins C and E. These antioxidant compounds have different mechanisms of action, such as the activation of antioxidant enzymes, metal chelation, blocking lipid peroxidation or eliminating superoxide radicals (Carocho, M., and Ferreira, I.C.F.R (2013). A review on antioxidants, prooxidants and related controversy: Natural and synthetic compounds, screening and analysis methodologies and future perspectives. Food and Chemical Toxicology, 51, 5-25).

When an excess of oxidant molecules is produced that cannot be neutralised by the antioxidant defence, an imbalance is generated that is called *oxidative stress.* This state is directly related to the appearance and development of chronic diseases such as cancer, diabetes, cardiovascular diseases or neurodegenerative diseases (García-Sánchez, A, Miranda-Díaz, A.G. and Cardona-Muñoz, E.G. (2020). The Role of Oxidative Stress in Physiopathology and Pharmacological Treatment with Pro- and Antioxidant Properties in Chronic Diseases. Hindawi, 1-16) and to the weakening of the immune system (Matsushita, M., Freigang, S. Schneider, C., Conrad, M., Bornkamm, G.W., and Kopf, M. (2015). T cell lipid peroxidation induces ferroptosis and prevents immunity to infection. J. Exp. Med., 212(4), 555-568). Excessive production and accumulation of ROS causes interactions with cellular structures, such as the modification of the bases of nucleic acids, producing mutagenesis and carcinogenesis. It also alters protein structures, through its inactivation and denaturation, and they act on lipids causing lipid peroxidation, the effect of which is to alter the integrity and functionality of the membranes of biological systems (Huang, M.Z., and Li, J.Y. (2020). Physiological regulation of reactive oxygen species in organisms based on their physicochemical properties. Acta Physiologica, 228, 1-16). Lipid peroxidation is a chain reaction that triggers the formation of reactive compounds such as malondialdehyde (MDA) or 4-hydroxynonenal (HNE), that form cross-links with proteins giving rise to advanced lipoxidation products (Ayala, A., Muñoz, M.F., and Argüelles, S. (2014). Lipid Peroxidation: Production, Metabolism, and Signaling Mechanisms of Malondialdehyde and 4-Hydroxy-2-Nonenal. Hindaw, 1-31).

For all these reasons, the current interest in obtaining compounds and formulations with antioxidant activity, including functional foods and ingredients, is obvious given its ability to provide health benefits through its intervention in physiological processes that promote the reduction of oxidative stress (Miguel, M., Vassallo, D. V., and Wiggers, G. A. (2020). Bioactive peptides and hydrolysates from egg proteins as a new tool for protection against cardiovascular problems. Current Pharmaceutical Design, 26(30), 3676-3683). A 2003 study showed that the consumption of ham produces a decrease of triglycerides in the plasma, blood pressure and lipid peroxidation, in addition to an increase in antioxidants, which reduces the atherogenic risk in the body (Mayoral, P., Martinez-Salgado, C.S., Santiago, J.M., Rodríguez-Hernández, M.V., Garcia-Gómez, M.L., Morales, A., López-Novoa, J.M. and Marcías-Nuñez, J.F. (2003). Effect of ham protein substitution on oxidative stress in older adults. Nutr Health Aging, 7(2), 84-9).

The *in vitro* determination of the antioxidant capacity of a compound or formulation, such as a food ingredient or a medicine, cannot be directly related to its *in vivo* antioxidant capacity, which is influenced by multiple factors such as stability, bioavailability and its distribution in tissues (intestine, vascular system and liver), which are barriers that these compounds have to overcome to reach their place of action in an active form. However, the possible influence of these parameters is not taken into account in the *in vitro* assays. Given the relevance that oxidative stress has taken on today, and the crucial role that antioxidants can play in health, their *in vivo* evaluation is necessary through the use of experimental models that also allow research into the possible mechanisms of action through which a bioactive compound or ingredient can exert its biological activity in the organism, at the cellular and molecular level (Huang, D., Ou, B., and Prior, R.L. (2005). The Chemistry behind Antioxidant Capacity Assays. J. Agric. Food Chem., 53, 1841-1856; MacDonald-Wicks, L.K., Wood, L.G., and Garg, M.L. (2006). Methodology for the determination of biological antioxidant capacity in vitro: a review. J Sci Food Agric, 86, 2046-2056).

In this sense, the use of biomarkers of lipid and protein oxidation, as well as the use of biomarkers of DNA damage, can be very suitable to evaluate and monitor changes in the redox system in *in vivo* situations. Lastly, it is necessary to check, by means of human trials, the effectiveness and safety thereof before its possible marketing that claims this biological effect (Morales, D., Miguel, M., and Garcés-Rimón, M., (2021). Pseudocereals: a novel source of biologically active peptides. Critical Reviews in food science and nutrition, 61 (9), 1537-1544). In fact, intervention studies in humans allow the optimal dose of the bioactive compound to be established, as well as the potential consumption risk in the general population and in certain groups (Vettorazzi, A., López de Cerain, A., Sanz-Serrano, J., Gil, A.G., and Azqueta, A. (2020). European Regulatory Framework and Safety Assessment of Food-Related Bioactive Compounds. Nutrients, 12, 613, 1-16). However, no studies have been found in which the *in vivo* antioxidant activity of Iberian pork fat and its health implications are analysed.

Moreover, current legislation on the use of test animals makes these *in vivo* tests socially, ethically and legally complicated (Sneddon, L.U., Halsey, L.G., and Bury, N.R. (2017). Considering aspects of the 3Rs principles within experimental animal biology. Journal of Experimental Biology, 220, 3007-3016). In fact, the 3 Rs (Replacement, Reduction and Refinement) published by Russel and Burch in 1959, which promotes the replacement of animal models with cells, tissues or computational models whenever possible (Sneddon, L.U., Halsey, L.G., and Bury, N.R. (2017). Considering aspects of the 3Rs principles within experimental animal biology. Journal of Experimental Biology, 220, 3007-3016), they are increasingly in demanded in research projects in Spain and in Europe. This is why, in recent years, numerous research projects have been carried out with the aim of finding alternatives for these test animals (especially for murine models and other mammals). In that sense, the use of yeasts as a model organism for determining the relevant biological activity of certain foods has been widely used in recent years as an option for carrying out *in vivo* studies. Specifically, it has been proven that the yeast *Saccharomyces cerevisiae* (*S. cerevisiae*) represents a viable alternative.

*S. cerevisiae* (BY4741) is a eukaryotic microorganism belonging to the unicellular fungal phylum Saccharomycotina. It is a model organism frequently used in the research of various processes related to human health such as, for example, oxidative stress and the effect of certain compounds on antibiotic resistance. The reasons for its use are very varied: it is an easy-to-use organism; it allows the study of millions of cells in low volume cultures; its genome has been widely studied, currently having one of the best functional annotations; the presence of orthologous genes and similarities at a physiological level with humans the results obtained in the trials to be extrapolated (Smith, M.G. and Snyder, M. (2006). Yeast as a Model for Human Disease. Current Protocols in Human Genetics, 48, 15.6.1-15.6.8; Bolotin-Fukuhara, M., Dumas, B., and Gaillardin, C. (2010). Yeasts as a model for human diseases. FEMS Yeast Res., 10, 959-960; Karathia, H., Vilaprinyo, E., Sorribas, A., and Alves, R. (2011). Saccharomyces cerevisiae as a model organism: a comparative study. PloS one, 6(2), 1-10; Pérez de Vega, M.J., Moreno-Fernández, S., Pontes-Quero, G.M., González-Amor, M., Vázquez-Lasa, B., Sabater-Muñoz, B., Briones, A.M., Aguilar, M.R., Miguel, M., and González-Muñiz, R., (2020). Characterization of Novel Synthetic Polyphenols: Validation of Antioxidant and Vasculoprotective Activities. Antioxidants, 9(787), 1-23). And above all, it represents an advantage in terms of ethical considerations, avoiding the use of animal models. It is worth mentioning that *S. cerevisiae* is one of the most used eukaryotic model organisms, having been used in studies on aging, regulation of gene expression, signal transduction and neurodegenerative diseases, among others (Karathia, H., Vilaprinyo, E., Sorribas, A., and Alves, R. (2011). Saccharomyces cerevisiae as a model organism: a comparative study. PloS one, 6(2), 1-10).

In the same way that cells have defence mechanisms, *S. cerevisiae* has transcription factors and enzymes directly involved in the response to oxidative stress such as superoxide dismutases (SODs), glutathione peroxidases (GPXs), catalases, etc., similar to those of mammals also involved in cellular aging (Takagi, H., and Kitagaki, H. (Eds.). (2015). Stress Biology of Yeasts and Fungi. Tokyo, Japan; Eleutherio, E., de Araujo Brasil, A., Braga França, M., Seixas Gomes de Almeida, D., Breves Rona, G., and Silva Magalhaess, R.S. (2018). Oxidative stress and aging: Learning from yeast lessons. Fungal Biology, 122, 514-525).

It has been seen that polyphenol-enriched cocoa presents *in vivo* resistance to oxidative stress in model organisms such as yeast *S. cerevisiae* or the worm *C. elegans* (Martorell, P., Forment, J.V., de Llanos, R., Montón, F., Llopis, S., González, N., Genovés, S., Cienfuegos, E., Monzó, H., and Ramón, D. (2011). Use of Saccharomyces cerevisiae and Caenorhabditis elegans as Model Organisms to Study the Effect of Cocoa Polyphenols in the Resistance to Oxidative Stress. J. Agric. Food Chem., 59(5), 2077-2085). Quercetin, one of the most abundant dietary flavonoids and one of the most studied antioxidants, has been used to estimate the effect on *in vivo* stress resistance of cells *S. cerevisiae,* taking the effect on its exponential growth as measurements. The results obtained suggest that quercetin in high concentrations can act as an antioxidant, providing partial protection to proteins against ROS-induced modification under exposure to thermal shock and oxidative stress (Bayliak, M.M., Burdylyuk, N.I., and Lushchak, V.I. (2016). Quercetin increases stress resistance in the yeast Saccharomyces cerevisiae not only as an antioxidant. Ann Microbiol., 66, 569-576). Meng et al. 2017, conducted a study that aimed to verify the antioxidant activity of natural dietary products (blueberries, resveratrol, catechins and quercetins, among others), using *S. cerevisiae* as a model system. In this study, it was concluded that many of the natural products studied are effective antioxidants in *S. cerevisiae* (Meng, D., Zhang, P., Li, S., Ho, C.T., and Zhao, H. (2017). Antioxidant activity evaluation of dietary phytochemicals using Saccharomyces cerevisiae as a model. Journal of Functional Foods, 38, 36-44). More recently, it has been shown that the flavonoid hyperoside (mainly present in fruits and vegetables) has a protective function in *S. cerevisiae* in response to oxidative stress induced in this unicellular organism (Gao, Y., Fang, L., Wang, X., Lan, R., Wang, M., Du, G., Guan, W., Liu, J., Brennan, M., Guo, H., Brennan, C., and Zhao, H. (2019). Antioxidant Activity Evaluation of Dietary Flavonoid Hyperoside Using Saccharomyces Cerevisiae as a Model. Molecules, 24(788), 1-12). All this indicates that the yeast *S. cerevisiae* is an effective model, and is more natural for the study of active antioxidant compounds and products, such as foods, and to assist and complement *in vivo* research (Meng, D., Zhang, P., Li, S., Ho, C.T., and Zhao, H. (2017). Antioxidant activity evaluation of dietary phytochemicals using Saccharomyces cerevisiae as a model. Journal of Functional Foods, 38, 36-44).

Given this possibility offered by *in vitro* experimental models with S. *cerevisiae* of oxidation mechanisms in the body, and given the deficiencies detected in this field of research, the present invention has been developed in order to offer a new drug, which is completely natural, in the treatment of diseases linked to oxidative stress.

**DESCRIPTION OF THE INVENTION**

The main object of the present invention is an extract derived from (originating from) pork ham fat, preferably from fat trimmings, and also preferably Iberian ham. Preferably, the ham is acorn-fed, and even more preferably from acorn-fed Iberian pigs, such as montanera pigs, an example being Joselito® pigs. In the most preferred case, ham fat is fat trimmings from pork hams such as those described, in any of its variants, with a minimum curing time of 36 months (i.e., equal to or greater than 36 months).

The main object of the present invention is also a pharmaceutical composition based on (comprising) an extract derived from (originating from) pork ham fat as an active ingredient, in an amount comprised between 75% and 95% by weight of the total weight of the composition, this amount preferably being between 92% and 95%, including both limits, and 95%, being the preferred amount. Likewise, the invention relates to the use in medicine of said pharmaceutical composition comprising pork ham fat extract as an active ingredient, preferably from fat trimmings, and also preferably Iberian ham. Preferably, it relates the use of the composition, and therefore the pork ham fat extract, for the prevention of diseases related to cellular aging, and even more preferably to the prevention of diseases such as cancer, diabetes, cardiovascular diseases, inflammatory bowel diseases or neurodegenerative diseases, among the most important, although additionally to those that are related to the weakening of the immune system; preferably being selected from cardiovascular diseases or inflammatory bowel diseases.

Similarly to plant extracts, which are widely known and extracted directly from the parts of the plant (fruits, leaves, seeds or roots), the present invention provides a composition that is an animal extract, extracted from pork fat, specifically from the fat of (cured) hams obtained therefrom. Said extract (which is obtained mainly, although in a non-limiting way, for nutritional purposes, i.e., they may sometimes be referred to herein as "food extract") contains components in its formulation, due to the very nature of the raw material, that perform a beneficial function in the body, which is useful in the prevention of diseases related to oxidative stress, thus meaning it is configured as a high-value food supplement (functional food). In fact, this extract is a food with great nutritional value, since, despite being considered a red meat, its consumption in the diet increases the intake of antioxidant substances and decreases lipid peroxidation, which generates beneficial effects in reducing atherogenic risk factors (cardiovascular diseases) (Mayoral, et al. 2003).

In the scope of the present invention, "pork ham" is understood as cured ham obtained from the hindquarters of the animal, and are subjected to seasoning and curing in particular environmental conditions: after applying salt over a few days, the salt is removed and it is hung to be exposed to the wind, with low temperatures and low humidity, without artificial drying or heating techniques. The curing process is maintained for at least 36 months, preferably 2 years. The fat is extracted from said piece of ham, the fat trimmings is the name given the fat covering the ham. This is a part of the ham that is not intended for direct consumption, but it is a by-product of great value because it maintains the nutritional and sensory characteristics of the product, so its use and revaluation is highly interesting, even from a sustainable point of view. The Iberian acorn-fed pig is one that, being of a variety of pig breed belonging to the so-called "tronco ibérico" or variety and predominant in the Iberian Peninsula, feeds on acorns and grass, which is known as the montanera period. Acorns have a high oleic acid content (56-58%) similar to that of olive oil, from which its high nutritional value is derived.

The extract produced is preferably in the form of fat, since it is made up of 100% fat from pork ham, such that it is in semi-solid form at room temperature (for example, between 18°C and 20°C) and liquid above 37°C, due to its content in monounsaturated fatty acids (MUFA) and polyunsaturated fatty acids (PUFA). However, in multiple embodiments, the extract can be presented in one of the forms selected from the group consisting of: dry, dehydrated, emulsified, diluted, lyophilised, atomised, and centrifuged extract, without altering the properties thereof, or included in culinary preparations such as creams, sauces, purées, ice cream, etc.

It should be noted that when a range of values is cited herein, its lower and upper limits are always included in the scope of the invention, as specific embodiments thereof.

The pork fat extract object of the present invention also has a very favourable fat content from a health point of view. Preferably, it has a content of one or more monounsaturated fatty acids comprised between 60% and 62% by weight with respect to the total weight of the extract (for example, of every 100 grams of extract obtained, between 60 and 62 grams are monounsaturated fatty acids), of which the oleic fatty acid is between 55% and 59% by weight of the total weight of the extract, more preferably between 56% and 58%, i.e., around 57% ($\pm$1% or 2%). This is relevant to the extent that, in addition to some valued organoleptic properties, which are essential in its use as a food condiment, the extract has properties that are beneficial for health, thanks to the antihypertensive effect and the ability to prevent cardiovascular diseases, associated in the literature with this fatty acid, becoming a very useful functional and nutraceutical supplement and, at the same time, appreciated for its flavour. Furthermore, the extract has antioxidant properties, measured using the ORAC (Oxygen Radical Absorption Capacity) method, using fluorescein as a fluorescent substance (ORAC-FL; Garc6s-Rim6n, M., González, C., Uranga J.A., López-Miranda, V., López-Fandiño, R., and Miguel, M. (2016). Pepsin Egg White Hydrolysate Ameliorates Obesity-Related Oxidative Stress, Inflammation and Steatosis in Zucker Fatty Rats. PLoS ONE 11(3):1-15), modified for a lipophilic sample. The extract has between 2 and 2.5 $\mu$mol equivalents of Trolox/g of extract, more preferably between 2.25 and 2.29 $\mu$mol equivalents of Trolox/g of extract, these values being higher than those obtained with certain oils, such as peanut oil or refined olive oil (Ninfali, P., Aluigi, G., Bacchiocca, M., and Magnani, M. (2001). Antioxidant Capacity of Extra-Virgin Olive Oils. JAOCS, 78(3), 243-247).

Continuing with the fatty acid content, the extract can have, in addition to the monounsaturated fatty acids described above, one or more saturated fatty acids (SFAs), in an amount comprised between 30% and 33% by weight with respect to the total weight of the extract; and one or more polyunsaturated fatty acids (PUFA), in an amount comprised between 7% and 10% by weight with respect to the total weight of the extract, more preferably 8%.

Among the saturated fatty acids are palmitic acid (between 19% and 21% by weight with respect to the total weight of the extract, the one with the greatest presence) and stearic acid (between 8% and 10.5% by weight with respect to the total weight of the extract), predominantly, although other acids may also appear in smaller quantities (the following are identified, in much smaller proportions, lauric acid, myristic acid and the margaric acid, for example). In turn, the monounsaturated fatty acids also include, in addition to oleic acid, palmitoleic acid (between 2%-2.5% by weight with respect to the total weight of the extract), eicosenoic acid (between 1% and 1.5% by weight with respect to the total weight of the extract) and, to a lesser extent, cis-10-heptadecenoic acid (equal to or less than 0.5% by weight with respect to the total weight of the extract). Regarding polyunsaturated fatty acids, these are mainly linoleic acid (between 6% and 9%) and $\alpha$-linolenic acid (between 0.5% and 1.5% by weight with respect to the total weight of the extract).

The degree of acidity of the pork ham fat extract can be between 23% and 26.5% by weight with respect to the total weight of the extract, more preferably between 24%-25%, according to official European analysis methods (Commission Implementing Regulation (EU) 2015/1833 of 12 October 2015 for oils); the acidity of oils is the result of the breakdown of triglycerides into glycerine and fatty acids, due to lipase enzymes.

A second object of the present invention is a production method for producing the pork ham fat extract described above, in any of its variants, which includes: heating (or in other words, increasing the temperature) of pork (ham) fat under vacuum conditions with rotation, at a pressure comprised between 55 and 930 mbar and at a temperature comprised between 30°C-90°C; with a rotation speed comprised between 80-90 rpm; and for an estimated time comprised between 40 minutes and 130 minutes, preferably between 45 minutes and 120 minutes.

This method produces, under vacuum conditions, an extract with the composition object of the invention without the use of solvents (i.e., in the absence of solvents), since a vacuum system is used (for example, a rotary evaporator) that reduces the internal pressure of the system. A pressure of 100 mbar is sufficient to extract the composition object of the invention, being established as the lowest recommended and preferable pressure that can be used to produce the extract. Preferably, the pressure is comprised between 90-120 mbar. In the most preferred case of all, the pressure value is between 100 and 120 mbar, being more preferably around 100 mbar.

Regarding the temperature used to produce the extract, this may preferably be comprised between 30°C and 85°C, more preferably between 30°C and 82°C, and even more preferably it can be selected from the group consisting of: around 31°C (i.e., understood as between 30-32°C), around 61°C (i.e., understood as between 60-62°C) and around 81°C (i.e., understood as between 80-82°C), being the most preferable of all, due to its properties, 60-62°C. A variation in temperature allows the properties of the extract to be modulated, changing its transparency, colour, brightness and organoleptic characteristics.

If all the variables analysed are taken into account, such as data relating to process performance, characterisation of fatty acid content, degree of acidity, *in vitro* antioxidant activity and consumer acceptance study, it can be concluded that the extract produced at a temperature between 55°C and 65°C, more preferably at 60°C, is the one that combines the best functional and sensory characteristics and properties.

According to this method, for every 1 kg of initial pork ham fat, 300-350 g of extract are produced, more normally 320 gr of extract, which shows high process performance.

Preferably, the vacuum heating step is carried out in a rotary evaporator, although it can be any device configured to rotate under vacuum conditions, and that can be heated to the temperatures required in this method.

Regarding the extraction time, it was found that this time varied depending on the temperature used: the lower the temperature, the longer the time needed to produce the same amount of extract. For example, at a temperature below 40°C (30°C or 37°C, for example), 2 hours of processing are required, while between 40°C and 70°C it takes half the time (1 hour); at 80°C or more, only 45 minutes are needed.

Once the extract is obtained, it can preferably be stored at a temperature between 2°C and -22°C, i.e., in refrigerated or frozen conditions, without altering the properties thereof and preserving them. Preferably, the extract can be kept under refrigerated (2-10°C) or frozen (-18 to -22°C) conditions for a maximum of 14 months under frozen conditions, and a maximum time of 8 months under refrigerated conditions, more preferably no more than 12 months frozen and more preferably no more than 6 months under refrigeration, without the properties thereof suffering degradation and without altering the microbiological properties thereof.

It is clear that any pork ham fat extract that can be produced from the process described above is part of the invention, in any of its variants. The extract may itself be a food composition, such as a food ingredient, and can be used as a seasoning and/or flavouring or as a functional food supplement, i.e., a nutraceutical supplement. Pork fat extract works directly as a functional food ingredient, meaning that it can be said that the nutritional supplement can be made up entirely (100% of the total composition of the supplement) of pork fat extract.

Likewise, part of the invention is a food or food product comprising in the formulation thereof the extract of interest, i.e., wherein the extract is accompanied by other food ingredients. Said food can be, for example, a meat product, a dairy product, such as fresh cheese, or a sauce, such as mayonnaise. Preferably, said food can be selected within the group consisting of: meat product, dairy product, dairy derivative, sauce, food paste, baking and pastry product or others, and any mixture thereof. Preferably, the food, depending on the group to which it belongs, may contain the extract in an amount comprised between 1% and 50% by weight of the total weight of the food. After different studies on the behaviour of the extract under different conditions or culinary preparation techniques, it has been proven that this can be subjected to, without degrading its properties, drying, dehydrating, lyophilisation, centrifuging, baking, freezing, airing, fermenting, boiling and stewing, producing new and different foods with pork ham fat extract. Furthermore, having performed rheology and texture tests, it has been seen that it has the appropriate rheological properties such as plasticity, viscosity, deformation capacity and texture capacity to be used in the culinary field.

Both the pork fat extract and the nutritional or food supplement that may comprise it, in any of its variants, can be packaged in bulk or in unit doses (depending on the doses required). Generally, the extract and food supplement can be packaged in cans, glass, drums, brick or in any other type of packaging known in the field.

In this sense, a fourth object of the invention is the use of the pork ham fat extract described above, in any of its variants, as a food composition, and this may preferably be a food (in itself) or a food ingredient. In addition, due to its antioxidant, antihypertensive and cardiovascular disease-preventive properties, the food composition can be used for the formulation of a functional food, i.e., a nutraceutical food. This food prevents coronary heart diseases, increases the intake of antioxidant components, and promotes the formation of antiinflammatory microbial flora in the intestine, preventing inflammatory bowel diseases, such as ulcerative colitis (Fernández, J., Garcia de la Fuente, V., Fernández Garcia, M.T., Gómez Sánchez, J., Isabel Redondo, B., Villar, C.J. and Lomb, F. (2020). A diet based on cured acorn-fed ham with oleic acid content promotes anti- inflammatory gut microbiota and prevents ulcerative colitis in an animal model. Lipids in Health and Disease, 1-19). The use of the extract is also envisaged for the manufacture of all the different functional products, food condiments and foods or food products envisaged herein, demonstrating great versatility.

In the scope of the present invention, it must be understood that the use in medicine of the pharmaceutical composition (or the extract that it includes as an active ingredient) essentially covers a product for use in medicine, and specifically for the prevention of the diseases mentioned in this document. Likewise, it indirectly relates to the use of the extract defined herein as an active ingredient for the manufacture of a pharmaceutical composition useful in the treatment of said (cardiovascular and inflammatory bowel) diseases.

This new medical use of the extract from pork ham fat was observed when analysing the *in vivo* antioxidant capacity in different samples of said extract as defined above in this document. To that end, strain BY4741 of S. *cerevisiae* was used as an experimental model, and its effect on improving cellular longevity and preventing cellular aging, said yeast being subjected to oxidative stress, using the mean $YPO_xD$ (with $H_2O_2$ at 0.01%) and the phenotypic effect of the application of each of the extracts was characterised. The effect of recovery of cellular activity (measured as recovery of the growth rate and rate of survivors or mass produced) promoted by the extract in the yeast S. *cerevisiae* was evaluated after exposure to a highly oxidising agent ($H_2O_2$) and this effect was compared to that produced by two recognised antioxidants such as vitamin C and resveratrol. In this sense, it was proven that the pork ham fat extract described herein has a positive effect on the cellular protection organism against oxidative stress since its lipid composition attributes antioxidant activity with a strong reduction in cell death caused by oxidative stress, and an increase in cell growth rate, which indicates recovery of redox balance and recovery from cellular damage caused by this phenomenon.

In that sense, the present invention offers the use in medicine of a composition comprising, as an active ingredient or agent, an animal extract, produced from pork fat, specifically from the fat of the (cured) hams obtained therefrom, in a similar way to plant extracts extracted from plants. Said extract contains components in the formulation thereof, due to the very nature of the raw material, that perform a beneficial function in the body, which is useful in the prevention of diseases related to oxidative stress, meaning it is configured as an active ingredient of great pharmaceutical value, since, as has been seen, its intake increases antioxidant substances in the body and decreases lipid peroxidation, which generates beneficial effects in reducing atherogenic risk factors (cardiovascular disease) (Mayoral, P.; Martinez-Salgado, C.; Santiago, J.; Rodriguez-Hernandez, M.; García-Gómez, M.; Morales, A.; López-Novoa, J.; Macías-Núñez, J. (2003). Effect of Ham Protein Substitution on Oxidative Stress in Older Adults. J. Nutr. Health & aging, 7(2), 84-89).

Preferably, the pork ham from which the extract that acts as the active ingredient is produced is acorn-fed, and even more preferably from acorn-fed Iberian pigs, such as montanera pigs, a very preferred example being Joselito® pigs. In the most preferred case of all, ham fat is fat trimmings from pork hams such as those described, in any of its variants, with a minimum curing time of 36 months (i.e., equal to or greater than 36 months).

Although, as said, the extract produced is preferably in the form of fat, by its very nature, in multiple embodiments, to be used as an active ingredient, the extract can be presented in one of the forms selected from the group consisting of: dry, dehydrated, emulsified, diluted, lyophilised, atomised, and centrifuged extract, without altering the properties thereof.

Also preferably, the extract that is used as an active ingredient has the content of one or more MUFAs as described previously herein and in the Spanish patent application P202131111, in any of its variants (i.e., all values and all variants and types specified above are applicable herein when referring to the extract as active ingredient). This is relevant to the extent that, in addition to having beneficial properties for health, thanks to the antihypertensive effect and the ability to prevent cardiovascular diseases, the extract presents pleasant organoleptic properties that make it easier for the patient to ingest, being appreciated for its flavour and aroma.

Regarding the saturated fatty acids (SFA) that the extract may contain, everything specified above when defining the extract applies herein, as well as the production method for producing it, and as specified in the priority Spanish patent application P202131111.

As mentioned earlier, the described pharmaceutical composition comprises pork fat extract as an active ingredient in an amount comprised between 75% and 95% by weight of the total weight of the composition, more preferably between 92% and 95% and, in the most preferred case of all, 95% by weight of the total weight of the composition.

Given its nature and its form, this extract is combined with excipients that allow it to be included and conserved in the pharmaceutical composition, which is ready to be ingested. In that sense, the pharmaceutical compound object of protection comprises, in any of its variants and regardless of the amount of active ingredient, one or more excipients or ingredients commonly used in the field of medicine, such as a mixture of tocopherols and/or plant oils (seed oil or coconut oil), and/or bulking agents, such as maltodextrin.

Preferably, the pharmaceutical composition described herein, in any of its variants, is used for the prevention of diseases, pathologies or conditions in which oxidative stress is involved, such as: cancer, diabetes, cardiovascular diseases, inflammatory bowel diseases, neurodegenerative diseases, dermatological diseases and those related to the weakening of the immune system. In the most preferred case of all, the pharmaceutical composition is used for the prevention of cardiovascular diseases and inflammatory bowel diseases (Mayoral, P.; Martinez-Salgado, C.; Santiago, J.; Rodríguez-Hernández, M.; García-Gómez, M.; Morales, A.; López-Novoa, J.; Macías-Núñez, J. (2003). Effect of Ham Protein Substitution on Oxidative Stress in Older Adults. J. Nutr. Health & aging, 7(2), 84-89; Fernández, J., Garcia de la Fuente, V., Fernández Garcia, M. T., Gómez Sánchez, J., Isabel Redondo, B., Villar, C.J., and Lombó, F., (2020). A diet based on cured acorn-fed ham with oleic acid content promotes anti- inflammatory gut microbiota and prevents ulcerative colitis in an animal model. Lipids in Health and Disease, 19(28), 1-19).

The daily dose to be administered of the active ingredient in preventive treatment is inferred from the daily intake of foods that can also act against oxidative stress. In that sense, said daily dose of active ingredient is preferably at least 0.5% of the daily intake of this type of extract. In a preferred case, the dose is specifically 0.5% of the daily intake, i.e., if we eat 100 g of foods per day that can also act against oxidative stress, the intake of the active ingredient would be 0.5 g; and in the case in which 95% of the pharmaceutical formulation is the active ingredient it would be 0.53 g. More preferably, the daily dose of extract to be ingested is between 0.5% and 1.5% of the total amount of the daily intake, including both limits.

## Brief Description of the Figures

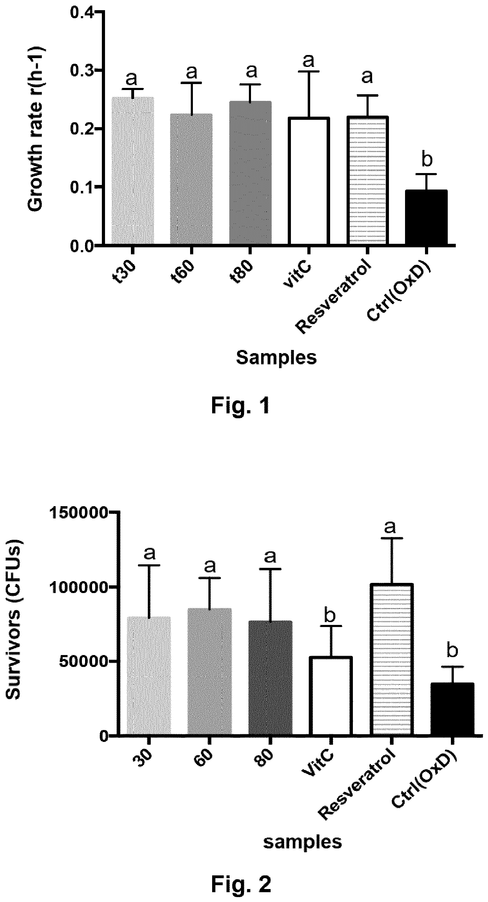

**Figure 1.** Growth rate of *Saccharomyces cerevisiae* subjected to oxidative stress (YPOxD) with different pork fat extracts according to the present invention, and as defined in patent application P202130236 (extracted at 30°C, 60°C and 80°C, and called extract t30, t60 and t90, respectively) and recognised antioxidant compounds (vitamin C -VitC- and resveratrol), as well as a control sample (Ctrl(OxD). Average of three measurements $\pm$ standard deviation. Different letters show significant differences (p <0.05).

**Figure 2.** Survivors as colony-forming units, CFUs per ml (mean $\pm$SD) of *Saccharomyces cerevisiae* subjected to oxidative stress (YPOxD) with the different pork ham fat extracts according to Example 1, and the other antioxidants analysed (vitamin C and resveratrol). Average of three measurements $\pm$ standard deviation. Different letters show significant differences (p <0.05).

## Exemplary embodiments

The results shown below are expressed as the mean $\pm$ standard deviation (SD) or as the mean $\pm$ standard error of the mean (SEM). The data were analysed using the one-way or two-way ANOVA statistical test where a value of p<0.05 indicates that there are significant differences between two analysed values.

## Example 1: Producing pork ham fat extract according to the present invention

Cárnicas Joselito S.L. prepared a quantity of 700 grams of ham subcutaneous fat for each Joselito® ham with a minimum curing time of 36 months. This ham marketed under the Joselito® brand is a cured Iberian ham that has been exclusively fed on acorns and grass during the montanera period.

To test the method and produce the extract, the fat was placed in a flask and rotated in a BÜCHI Labortechnik AG, 2019, rotary evaporator under the following conditions, combined in different ways, to check the extraction performance (Table 1):

- a temperature of: 30°C, 37°C, 45°C, 60°C, 70°C, 80°C and 90°C;
- a pressure selected between: 60 mbar, 100 mbar, 400 mbar, 800 mbar and 930 mbar;
- a stirring speed comprised between 80-90 rpm.

**Table 1. Conditions for producing the pork fat extract and characteristics of the samples produced after placing the Joselito® ham fat under vacuum**

| Temp (°C) | Stirring Speed (rpm) | Pressure (mbar) | Incubate d sample (gr) | Stirring time | Extracte d sample (ml) | Aliquots stored under refrigeration | Aliquots stored frozen |
|---|---|---|---|---|---|---|---|
| 30 | 80-90 | 60 | 20 | 2 hours | 20 | 5x2 ml | 5x2 ml |
|  |  | 100 |  |  | 20 | 5x2 ml | 5x2 ml |
|  |  | 930 |  |  | 20 | 5x2 ml | 5x2 ml |
| 37 | 80-90 | 60 | 20 | 2 hours | 20 | 5x2 ml | 5x2 ml |
|  |  | 100 |  |  | 20 | 5x2 ml | 5x2 ml |
| 45 | 80-90 | 60 | 20 | 1 hour | 20 | 5x2 ml | 5x2 ml |
|  |  | 100 |  |  | 25 | 5x2 ml | 7x2 ml |
| 60 | 80-90 | 60 | 20 | 1 hour | 20 | 5x2 ml | 5x2 ml |
|  |  | 100 |  |  | 25 | 5x2 ml | 7x2 ml |
|  |  | 400 |  |  | 25 | 5x2 ml | 7x2 ml |
|  |  | 800 |  |  | 25 | 5x2 ml | 7x2 ml |
|  |  | 936 |  |  | 25 | 5x2 ml | 7x2 ml |
| 70 | 80-90 | 60 | 20 | 1 hour | 25 | 5x2 ml | 7x2 ml |
|  |  | 100 |  |  | 30 | 5x2 ml | 10 × 2 ml |
| 80 | 80-90 | 60 | 20 | 45 min | 25 | 5x2 ml | 7x2 ml |
|  |  | 100 |  |  | 30 | 5x2 ml | 10 × 2 ml |
| 90 | 80-90 | 60 | 20 | 45 min | 25 | 5x2 ml | 7 × 2 ml |
|  |  | 100 |  |  | 30 | 5x2 ml | 10 × 2 ml |

550 g of subcutaneous ham fat were required to extract 250 ml of extract.

Subsequently, several aliquots were obtained that were stored at different temperatures comprised between 2°C and -22°C in order to check how the manner of storage affected the sample. This step is detailed in Example 7, to determine the stability and useful life of the extract.

The results after the process of producing the extract under the vacuum conditions in the rotary evaporator defined herein, subjecting the fat samples to the established temperatures (30°C, 37°C, 45°C, 60°C, 70°C, 80°C, 90°C), are shown in Table 2.

Initially, a preliminary study was carried out using different pressures in the rotary evaporator (Table 2) and it was finally established that a flask pressure of 100 mbar was sufficient to achieve the extraction of the essence.

**Table 2. Extracts object of the invention produced under vacuum conditions using rotary evaporation rotational evaporation)**

| Temp. | Extraction time | Fat colour | Fat texture | Extract colour | Extract smell |
|---|---|---|---|---|---|
| 30°C | 2 hours | Pinkish | Excess hardness, fibrous | Yellowish, compact | Dry, bitter |
| 37°C | 2 hours | Pale yellow | Fibrous and gelatinous | Less opaque/ compact | Less intense, pleasant |
| 45°C | 1 hour | Bright | Fibrous | Very viscous | Intense, overpowering |
| 60°C | 1 hour | Toasted, bright | Hard | Yellow | Not bitter, moderate intensity |
| 70°C | 1 hour | Pale yellow, toasted | Gelatinous | Light yellow, less opaque | Non-irritating, moderate intensity |
| 80°C | 45 min | Brown, toasted | Soft | Light yellow | Pleasant |
| 90°C | 45 min | Pale yellow, toasted | Very soft | Light yellow, no lumps | Intense, rancid |

It is important to note that, after analysing the results of the extraction carried out under vacuum with the rotary evaporator, it is found that an increase in temperature causes a change in the properties of the extract: it becomes less opaque and shows a brighter, yellower colour.

**Example 2: Evaluation of the organoleptic and sensory properties of the samples of the extract produced in Example 1**

First, the basic organoleptic aspects were evaluated (appearance, colour, smell, texture and flavour) of both the raw material (pork fat trimmings) and the extract produced, to make a first discriminatory classification of the resulting products, and those that had unpleasant flavours and smells were discarded. In this analysis, the different people who make up the different research groups and the company Cárnicas Joselito, S.L. participated as evaluators in the project.
In that sense, the extracts produced under vacuum and with the different indicated temperatures were evaluated. The samples with the best results in terms of their basic sensory conditions were those that had been extracted at 30°C, 60°C and 80°C. Specifically, the extract produced at 30°C were delicate in the mouth, but the sample at 60°C had a greater ham flavour and smell (more intense). Moreover, the sample extracted at 80°C exhibited some bitterness, but it was pleasant in the mouth and had the aroma of a "ham cellar/drying room". According to the results obtained, it was decided to choose the samples extracted at 30°C, 60°C and 80°C to carry out the following analyses, due to the excellence of its properties.

**Example 3: Characterisation of the extract formulation and its fatty acid content**

The fatty acid analysis of the extracts produced was carried out according to the methodology described by Isabel, B. et al., 2014 (Isabel, B., Cordero, G., Olivares, A., Daza, A. and Lopez-Bote, C.J. (2014). Differential response of Iberian and lean pig crossbreeds to dietary linoleic acid administration. Spanish Journal of Agricultural Research, 12(2): 419-426), based on the acid extraction and methylation of methyl esters of fatty acids, for the subsequent injection and determination by gas chromatography thereof. Fatty acids were identified by gas chromatography using a Hewlett Packard 6890 gas chromatograph and a 30 m $\times$ 0.32 mm $\times$ 0.25 $\mu$m cross-linked polyethylene glycol capillary column. A temperature program of 170 to 245°C was used. The injector and detector were maintained at 250°C. The carrier gas (helium) flow rate was 2 ml/min. This is the methodology that Cárnicas Joselito S.L. regularly uses to analyse the quality of their hams.
First, the fatty acid (FA) composition was characterised. The three selected and extracted samples were analysed under vacuum conditions at different temperatures (30, 60, 80°C). Table 3 shows the results obtained from saturated (SFA), monounsaturated (MUFA) and polyunsaturated (PUFA) fatty acids for the different extract samples analysed.

**Table 3. Fatty acid composition of the extracts selected in Example 2 (according to the sensory properties thereof)**

| | | Extraction temperature | | |
|---|---|---|---|---|
| | | 30°C | 60°C | 80°C |
| SFA (%)* | Lauric acid | 0.06±0 | 0.05±0.01 | 0.05±0.01 |
| | Myristic acid | 1.10±0.03 | 1.06±0.09 | 1.05±0.08 |
| | Palmitic acid | 20.25±0.63 | 19.20±0.80 | 20.54±1.10 |
| | Margaric acid | 0.22±0.03 | 0.24±0.05 | 0.23±0 |
| | Stearic acid | 8.6±0.28 | 9.32±0.20 | 10.31±1.12 |
| | Arachidonic acid | 0.13±0.01 | 0.15±0.01 | 0.16±0.03 |
| | **Total SFA (%)** | **30.36** | **30.02** | **32.34** |
| MUFA (%)* | Palmitoleic acid | 2.16±0.03 | 2.14±0.20 | 2.12±0.14 |
| | Cis-10-heptadecenoic acid | 0.28±0.02 | 0.38±0.07 | 0.27±0.03 |
| | Oleic acid | 58.12±0.25 | 57.62±0.90 | 56.96±1.78 |
| | Eicosenoic acid | 1.26±0.04 | 1.8±0.07 | 1.25±0.08 |
| | **Total MUFA (%)** | **61.82** | **61.94** | **60.6** |
| PUFA (%)* | Linoleic acid | 7.17±0.10 | 7.39±0.42 | 6.51±0.43 |
| | $\alpha$-linolenic acid | 0.65±0.07 | 0.65±0.12 | 0.55±0.08 |
| | **Total PUFA (%)** | **7.82** | **8.04** | **7.06** |
| *Percentage with respect to the total weight of the extract produced* | | | | |

The obtained results mainly showed a drastic decrease in polyunsaturated fatty acids with increasing temperature in the extraction process. Moreover, a high percentage of oleic acid appears in all samples (56-58%), which gives it a potential antihypertensive and preventive effect on cardiovascular diseases.

**Example 4: Determination of the degree of acidity in the extract**

To determine the degree of acidity of the different samples of ham extract produced after the different treatments described in the previous examples, official European analysis methods were used (Commission Implementing Regulation (EU) 2015/1833 of 12 October 2015, on the characteristics of olive oil and olive-residue oil and on the relevant methods of analysis). The determination of both parameters included in the Regulation, is applicable to animal and plant oils and fats. The two determinations (one per parameter) were carried out in triplicate of each of the extract samples in liquid form.

Table 4 shows the degree of acidity of each of the extracts.

**Table 4. Degree of acidity of the selected extracts**

| Extraction temperature | Degree of acidity |
|---|---|
| **30°C** | 24.74±1.58 |
| **60°C** | 24.18±0.34 |
| **80°C** | 25.89±0.76 |

As mentioned earlier, the degree of acidity allows the free acids in oils to be determined (Commission Implementing Regulation (EU) 2015/1833). According to current regulations, extra virgin olive oil and virgin olive oil must have an acidity level of less than 0.8-2, and thus the results obtained herein for ham fat extracts would be much higher than this limit. When compared with other oil of plant origin, such as avocado oil, it can be seen that the values are also higher (1.29 ± 0.02% of oleic acid) (Ariza, J.A., López., F., Coyotl., J., Ramos, ME., Diaz, J., and Martinez, A. (2011). Effect of different extraction methods on the fatty acid profile in the avocado (Persea americana Mill. var. Hass) oil. Venezuelan Magazine of Food Science and Technology, 2 (2): 263-276).

Example 5: Measurement of *in vitro* antioxidant activity of the pork ham fat extract

With the aim of knowing whether producing the extract through vacuum conditions at different temperatures influenced their antioxidant capacity, said antioxidant capacity of each of the selected samples was evaluated using the ORAC method (Garcés-Rimón, et. al., 2016). All the reagents used in the assay were diluted in phosphate buffer (PBS 75 mM, with a pH of 7.4). 6-Hydroxy-2,5,7,8-tetramethylchroman-2-carboxylic acid (Trolox; Sigma-Aldrich, Madrid, Spain) was diluted to obtain different concentrations (2.5-20 μg/ml) with which a reference calibration curve was constructed. Each sample (in triplicate) and the Trolox curve (in duplicate) were incubated (10 minutes, 37°C) with disodium fluorescein solution (116.61 nM, Sigma-Aldrich). After this incubation, 2,2'-azobis-(2-methylpropionamidine) dihydrochloride AAPH (48 mM, Sigma-Aldrich). The reaction was carried out in black polystyrene 96-multiwell plates (Corning, Kennebunk, United States) for 95 minutes at 37°C. Fluorescence was measured continuously throughout the reaction time in a fluorometer (Fluostar Optima, BMG Labtech, Heathfield, England), using an excitation wavelength of 485 nm and emission wavelength of 520 nm. Based on the resulting curves, the area under the curve (AUC) of fluorescence decay was calculated using the following formula:

$$AUC = 1 + \sum_{i=1}^{i=104} \frac{f_i}{f_0}$$

Where f0 is the initial fluorescence reading and fi is the fluorescence reading after i minutes. The AUC values of all the samples were normalised with respect to a blank (without antioxidant) and lines were prepared for each sample with different dilutions, where AUC is plotted on the Y-axis, and the concentration of each point on the X-axis. The slope obtained from each graph is divided by the slope of the calibration line.

Extraction of the lipophilic fraction was carried out with 80% methanol. Each of the samples along with 80% methanol were vigorously stirred for 2 min and then on a stirring plate without temperature for 15 min. Next, it was centrifuged at 18°C and 3000 g for 10 min, and the supernatant was recovered. Lastly, the collected supernatant was diluted in phosphate buffer (PBS 75 mM, with a pH of 7.4) at different concentrations.

All the samples were measured in triplicate and the test was repeated twice, using samples from different extractions. The results were expressed as μmol Trolox equivalent/g of extract. The results of the antioxidant capacity are shown in Table 5.

Table 5. Antioxidant capacity (μmol Trolox equivalent/g) of selected pork ham fat extracts

| Extraction temperature | Antioxidant capacity (μmol Trolox equivalent/g) |
|---|---|
| 30°C | 2.25±0.01 |
| 60°C | 2.27±0.09 |
| 80°C | 2.29±0.01 |

No differences were observed in terms of antioxidant capacity between the three extracts selected for the analysis. These results suggest that the extraction temperature to which pork ham fat is subjected is not a determining factor in the antioxidant capacity thereof since no significant differences were found between them.

The results of the antioxidant capacity of extracts obtained from pork ham fat, specifically from Joselito[®] Iberian pork, are lower than the results obtained in different types of extra virgin olive oil samples (3.60 - 8 μmol Trolox equivalent/g) (Ninfali et. al., 2001); but they are higher than those obtained from other oils such as peanut oil, and also higher than the antioxidant capacity of refined olive oil (1.06 ± 0.09, 1.55 ± 0.08, respectively) (Ninfali, et.al., 2001). More recently, other plant oils have been studied with lower results than those obtained in the different extracts obtained from pork ham fat, specifically Joselito[®] Iberian pork ham (2.25-2.29 μmol Trolox equivalent/g). The results obtained in virgin coconut oil were 0.008 μmol Trolox equivalent/g (Ngampeerapong, C., Chavasit, V., and Durst, R.W. (2018). Bioactive and nutritional compounds in virgin coconut oils. Mal J Nutr, 24(2), 257-267), in soybean oil they were 0.091 ± 2.0 μmol Trolox equivalent/g (Giuffrè, A.M., Caracciolo, M., Zappia, C., Capocasale, M., and Poiana, M. (2018). Effect of heating on chemical parameters of extra virgin olive oil, pomace olive oil, soybean oil and palm oil. Ital. J. Food Sci., 30, 715-739) and in palm oil they were 0.654 ± 0.5 μmol Trolox equivalent/g (Giuffrè et. al., 2018).

**Example 6: Study of consumer acceptance of the extracts object of the present invention**

An acceptability study was carried out using a hedonic test (Thimoteo, D., Braz, R., Ribeiro, R., de Lacerda, L. and Stedefeldt, E. (2013). Methods for applying the tests of acceptability for school feeding: the validation of playful cards. Rev Chil Nutr, 40 (4), 357- 363). Taking into account the mobility and distancing restrictions caused by the current COVID-19 pandemic, in this study, the recruitment of participants was carried out electronically and, once the different samples to be evaluated were obtained, they were sent frozen to the home of the recruited participants, along with some basic instructions and a questionnaire. Participants also completed a survey aimed at their preferences when purchasing and consuming similar products.

In this study, the participants sensory evaluated 3 samples, extracted at different temperatures within the range 30-80°C and that were specifically obtained at 30°C, 60°C and 80°C, all of them with a vacuum pressure of 100 mbar, that were selected as a result of the previous tests of the basic sensory analysis.

The acceptability tests were carried out in various Spanish cities, with an age range between 18 and 70 years old, recruiting 33 participants who were not allergic to ham and/or its fat, and who preferably like this product. Among the total number of participants, 50% were women and the other 50% were men. For each of the samples, the results of the participants were averaged.

The general visual evaluation of the three samples obtained a similar result. The olfactory evaluation of the samples was superior in the samples extracted at 60°C and 80°C and the one that obtained the best result was the sample obtained at 60°C.

When analysing the changes or modifications that the study participants would introduce into the samples, it can be seen that 42.3% would not modify the sample at all, which indicates a high degree of acceptance of the product.

Once all data regarding performance, characterisation of fatty acids, degree of acidity, *in vitro* antioxidant activity and acceptance study has been collected and analysed, it was concluded that the sample extracted at 60°C was the most promising and the one that combined the best functional and sensory characteristics and properties, and therefore it was selected to carry out the determination of stability and useful life.

**Example 7: Determination of the stability and useful life of the extract object of the present invention, under refrigeration and after freezing**

A microbiological study was carried out to evaluate the changes produced in the selected extract samples, under refrigeration (2 to 10°C) and frozen (-18 to -22°C) under different storage periods and under regulated conditions (control of airtight storage of the samples and adaptation of the temperature within the established margins). Reference microorganisms (aerobic and anaerobic mesophiles, spores of aerobic and anaerobic microorganisms, moulds and yeasts, *Salmonella, Escherichia coli* and *Listeria monocytogenes*) were analysed and it was considered that when the analysis detected a quantity greater than that established in Regulation (EC) No. 1441/2007 in a product, the extract had reached the end of its useful life. The maximum values included in the Regulation are shown in Table 6.

**Table 6. Maximum permitted microbiological values (Regulation (EC) No. 1441/2007)**

| Microorganism analysed | Detection limit |
|---|---|
| *Salmonella* | Absence in 25 gr |
| Fungi and yeast | $10^2$ cfu/gr |
| *Listeria monocytogenes* | Absence in 25 gr |
| *Escherichia coli* | < 10 cfu/gr |
| Aerobic mesophiles | $10^5$ cfu/cm$^2$ |
| Spores of aerobic and anaerobic microorganisms | $10^2$ cfu/gr |

As indicated in Example 6, in order to carry out the stability and useful life study, the sample at 60°C, which was the most widely accepted, was used in aseptic conditions to ensure the final results of the analysis. To that end, it was necessary to sterilise all the material necessary for the extraction and work in a biosafety hood. The final sample volume necessary for the analysis of all the aforementioned parameters over time was 1500 ml. The sample was divided into aliquots for storage under refrigeration (2 to 10°C) and frozen (-18 to -22°C). The initially established analysis times may be modified according to previous results, the final objective being to check the stability of the product after 1 year of conservation. Furthermore, a sample will be necessary that is taken as time 0 to know the initial stability of the sample. The extraction process and the provisionally collected aliquots are listed below:

- Storage of fresh extract samples (2 to 10°C) for different times: 1 week, 1 month, 2 months, 4 months and 6 months;

- Storage of frozen extract samples (-18 to -22°C) for different times: 2 months, 4 months, 6 months, 8 months and 12 months.

A microbiological study was carried out in duplicate at zero extraction time with the extract obtained at 60°C in order to compare the microbial burden that the product initially presents. The results obtained after the analysis of the indicated microorganisms were within the maximum parameters established in the current regulations, thus, the product was considered microbiologically safe at zero extraction time.

In order to know the refrigeration stability of the extract obtained at 60°C, the microbiological studies described in the previous paragraph were carried out on the samples. First, the extract was analysed after 1 week under refrigeration. Moreover, a thawed extract sample that was kept refrigerated for 3 days was analysed. Once again, both samples exhibited great stability since the growth of the analysed microorganisms was not observed. After a month under refrigeration, the sample was reanalysed and a thawed sample that was kept refrigerated for 1 week was also analysed, and no growth of microorganisms was observed either.

Next, a new microbiological analysis was carried out after 6 months of freezing, and again no microbial growth was observed and the results obtained were within the parameters established in the current regulations.

These results suggest that the samples are stable and safe over time, since the results obtained show that there has been no growth of any microorganism and they are within the limits established in current legislation. Furthermore, the initial results (sample=time 0) obtained suggest that trimming the fat, preparing, packaging and shipping, as well as the process of producing the extract were carried out in the necessary aseptic conditions so that the initial microbiological burden of the sample is not above the reference values, and stability can be achieved over time.

No previous studies have been found in which the microbiological burden of a similar product is analysed.

**Example 8: Study of the determination of *in vivo* antioxidant activity of the pork ham extract used as active ingredient in the pharmaceutical composition object of the present invention**

Definition of the extract samples under study

Three extracts obtained at different temperatures were tested, 30°C, 60°C and 80°C, i.e., resulting from three different extraction processes from Joselito® brand acorn-fed Iberian pork ham fat. This ham is a cured Iberian ham that has been exclusively fed on acorns and grass during the montanera period. The fat used is subcutaneous ham fat from sliced Joselito® ham with a minimum curing time of 36 months. These samples were introduced into a BÜCHI Labortechnik AG, 2019 rotary evaporator under different conditions detailed below:

- a temperature of: 30°C, 60°C and 80°C;
- a pressure selected from: 100 mbar;
- a stirring speed comprised between 80-90 rpm.

In this way, 3 different samples were obtained from the same initial Joselito ham fat. The specific extraction conditions are contained in Table 7.

**Table 7. Conditions for producing the extracts under analysis**

| Nomenclature of extract samples produced | Extraction temperature (°C) | Pressure (mbar) | Stirring speed (rpm) | Extraction time |
|---|---|---|---|---|
| t30 | 30 | 100 | 80-90 | 2 hours |
| t60 | 60 | 100 | 80-90 | 1 hour |
| t80 | 80 | 100 | 80-90 | 45 min |

The three extracts obtained (samples t30, t60 and t80) have a high MUFA, antioxidant and phenolic compound composition. The detailed composition of fatty acids of the samples (t30, t60 and t80) is contained in Table 8, expressed in %.

**Table 8. Fatty acid composition of the analysed samples**

| Fatty acid (%) | Starting material | Extraction temperature (°C) | | |
|---|---|---|---|---|
| | | t30 | t60 | t80 |
| Lauric acid | 0.05±0 | 0.06±0 | 0.05±0.01 | 0.05±0.01 |

(continued)

| Fatty acid (%) | | Starting material | Extraction temperature (°C) | | |
|---|---|---|---|---|---|
| | | | t30 | t60 | t80 |
| | Myristic acid | 0.94±0.07 | 1.10±0.03 | 1.06±0.09 | 1.05±0.08 |

| Fatty acid (%) | | Starting material | Extraction temperature (°C) | | |
|---|---|---|---|---|---|
| | | | t30 | t60 | t80 |
| SFA (%) | Palmitic acid | 19.67±0.26 | 20.25±0.63 | 19.20±0.80 | 20.54±1.10 |
| | Margaric acid | 0.20±0.02 | 0.22±0.03 | 0.24±0.05 | 0.23±0 |
| | Stearic acid | 7.56±0.49[a] | 8.6±0.28[b] | 9.32±0.20[b,c] | 10.31±1.12[c] |
| | Arachidic acid | 0.15±0.01 | 0.13±0.01 | 0.15±0.01 | 0.16±0.03 |
| | **Total SFA (%)** | **28.56±0.51[a]** | **30.36±0.32[b]** | **30.02±0.91[b]** | **32.34±2.21[c]** |
| MUFA (%) | Palmitoleic acid | 1.95±0.09 | 2.16±0.03 | 2.14±0.20 | 2.12±0.14 |
| | Cis-10-heptadecenoic acid | 0.23±0.02 | 0.28±0.02 | 0.38±0.07 | 0.27±0.03 |
| | Oleic acid | 60.48±1.0[a] | 58.12±0.25[b] | 57.62±0.90[b] | 56.96±1.78[b] |
| | Eicosenoic acid | 1.46±0.06 | 1.26±0.04 | 1.8±0.07 | 1.25±0.08 |
| | **Total MUFA (%)** | **64.11±0.95[c]** | **61.82±0.22[b]** | **61.94±0.70[b]** | **60.6±1.77[a]** |
| PUFA (%) | Linoleic acid | 6.74±0.41 | 7.17±0.10 | 7.39±0.42 | 6.51±0.43 |
| | α-linolenic acid | 0.58±0.08 | 0.65±0.07 | 0.65±0.12 | 0.55±0.08 |
| | **Total PUFA (%)** | **7.33±0.48** | **7.82±0.17** | **8.04±0.53** | **7.06±0.51** |

*Average of three measurements ± standard deviation. Different letters in the same row show significant differences ($p < 0.05$).

In turn, the content of antioxidants and phenolic compounds (total phenolic content, or TPC) of the extracts is shown in table 9, expressed in μmol Trolox equivalent/g and in mg gallic acid equivalent/100 g, respectively.

**Table 9. Antioxidant capacity and phenolic compounds content of the analysed samples**

| Extraction temperature (°C) | Antioxidant capacity (μmol TE /g) | TPC (mg GAE / 100 g) |
|---|---|---|
| Starting material | 1.86±0.15[a] | 73.46±2.23[a] |
| t30 | 2.32±0.08[b] | 94.04±3.98[b] |
| t60 | 2.36±0.06[b] | 139.51±4.84[c] |
| t80 | 2.11±0.09[a,b] | 126.79±3.57[d] |

*Average of three measurements ± standard deviation. Different letters in the same column show significant differences ($p < 0.05$).

Furthermore, it was possible to verify that the extracts were very stable, since they could be stored for a period of 14 months under frozen conditions, and a period of 8 months under refrigerated conditions, without the properties thereof suffering degradation and without altering the microbiological properties thereof.

Characterisation of the antioxidant activity measurement method

The strain BY4741 of *S. cerevisiae* was used to determine the *in vivo* antioxidant activity. This strain was subjected to oxidative stress and continuous growth was monitored, with *Bioscreen c* (Oy Growth Curves Ab Ltd, Finland). This equipment allows you to continuously determine the optical density (OD) of a microculture on a large scale, uses exclusive plates called Honeycomb 10x10 wells with a maximum volume of 300 μl, and allows the use of two plates per run, testing 200 samples at a time.

For the growth of the BY4741 strain of *S. cerevisiae* a glycerine maintained at -80°C was used, inoculating 20 μl of the glycerine in 5ml of YPD medium (control medium: 2% yeast extract, 1% peptone and 2% glucose), for 24 h at 200 rpm and 28°C. From this pre-culture, it was subjected to oxidative stress by growing 500 μl in 5 ml of YPOxD medium (stress medium: 2% yeast extract, 1% peptone, 1% glucose and 0.01% $H_2O_2$), incubating for 16-24 h at 200 rpm and 28°C. Once an OD600 ~ 0.6 is reached (between 16 and 24h), it was ready to begin the characterisation of the antioxidant effect of the extracts analysed with the instrument *Bioscreen c*. To that end, the stressed preculture was diluted with YOPxD medium to obtain an OD600 ~ 0.1, and 180 $\mu$l of this yeast dilution were deposited in each well of the Honeycomb plate, using a multi-dispensing pipette (Eppendorf Multipette). Each sample was tested at four doses (1$\mu$l, 5$\mu$l, 10$\mu$l and 20$\mu$l), with at least 5 repetitions (technical replications) per dose. Vitamin C and resveratrol were used as comparative compounds of known antioxidant activity, and were tested at two doses (2$\mu$l and 10$\mu$l; 2mg/ml in 50% methanol) with at least 7 repetitions per dose. In each experiment, the corresponding negative controls are included (uninoculated media: YPD, YPOxD, YPOxD + 50% methanol) and stress culture controls (yeast under stress in YPOxD + 50% methanol), with at least 3 repetitions of each, being distributed between the two plates of the experiment. The assay was performed in a final volume of 200 $\mu$l.

The plates were incubated in the Bioscreen c under the following parameters: 200 samples; 600 nm filter, Brown; 28°C; experiment length of 5 days + 1 hour; OD600 measurements every 15 minutes; high and continuous stirring. The Bioscreen c collected OD600 data at 15 minute intervals. OD600 values were normalised to the mean value of the corresponding blank (negative controls). The growth rate (r) and burden capacity (k) parameters of the system were determined with the Growthcurver v.0.3.1 library in RStudio v. 1.4.1106 (Sprouffske & Wagner, 2016; RStudio 2021). This library adjusts the OD600 data to a logistic growth model, from which it determines the parameters that define the growth curve as r (growth rate, in h-1) and k (maximum population size or burden capacity), among others. This software also allows the presence of outliers to be checked through PCA analysis, using the Dplyr 1.0.6 and Ggplot2 3.3.3 packages (Sprouffske, K., and Wagner, A. (2016). Growthcurver: an R package for obtaining interpretable metrics from microbial growth curves. BMC Bioinformatics, 17(172), 2-4). Lastly, a file was generated with the characterisation data of the adjustment of the growth curve to the logistic model, for each of the technical replicas of each sample tested.

Once the experiment is finished, the number of viable cells was determined after exposing the samples to each of the extracts used as active pharmaceutical compound, as well as vitamin C, resveratrol and controls. For this, the number of colony-forming units (CFUs) was determined using the serial dilution technique and sowing in sterile 96-well plates. Serial dilution was performed with an automatic multichannel pipette (Eppendorf Xplorer® plus) transferring 20 $\mu$l from row A to the next row. Each row contained 180 $\mu$l of sterile 10% glycerol, performing homogenisation and transfer at the same time. Once each serial dilution plate is prepared, sowing took place in a Petri dish with YPDA medium (YPD agar) with the help of an 8x6 pin replicator (SIGMA replica plater for 96 well plate, 8x6 array, ref. R2383-1EA) sterilised by flame with 96% ethanol. This replicator deposited 1.65 $\mu$l from each well into each Petri dish. Three technical replicates of each seeding were carried out in a Petri dish. The plates were kept in a face-up position and incubated for 48 h at 28°C. The number of colonies obtained in each replicate was determined, and by correcting with formula 1, the number of CFUs per ml is determined.

$$\text{CFUs/ml} = (\text{number of colonies x dilution factor}) / (1.65 \times 10^{-3})$$

Statistical Analysis

Through the program *Graphpad PRISM v6* it was determined if there were significant differences between the mean values of growth rate (r), burden capacity (k) and survivors (measured as CFUs/ml), using one-way ANOVA, with Tukey correction for multiple comparisons, establishing the level of significance with a value of $p < 0.05$. Data were plotted as mean $\pm$ SD, Indicating the level of significance.

Results and discussion on antioxidant effect and dose-response application

In the results obtained, it can be seen that oxidative stress negatively affected the growth parameters of the yeast, especially the growth rate (r) (Figure 1) and the survivors (Figure 2). It was observed that all samples of pork ham fat extract analysed provided greater recovery of cell growth parameters. No significant differences were observed between the samples analysed in terms of growth rate (r) and burden capacity (k) (burden capacity data not shown). All analysed samples of the extract, and antioxidant controls, vitamin C and resveratrol, showed a significant increase in growth rate compared to the OxD control (Figure 1). No significant differences were observed between the extract samples analysed and the antioxidant controls vitamin C and resveratrol (Figure 1). Regarding the results obtained in the survival rate (CFUs) in the extract samples, no significant differences were observed between them and resveratrol. Only vitamin C did not show a significant increase with respect to the OxD control (Figure 2).

These results demonstrate that the pork fat extract described here has *in vivo* antioxidant activity, since it allowed a notable recovery of biomass produced, growth rate and the number of viable cells (survivors), compared to the control sample subjected to oxidative stress. It is worth mentioning that the antioxidant effect of the extract was similar to that of resveratrol and even improved the effect on the number of viable cells (survivors) with respect to vitamin C by 31-38% (Figure 2), there being two compounds with recognised antioxidant effects. To that end, it can be concluded that the extract has a positive effect on the cellular protection organism against oxidative stress, which is related to the immune system: under situations of oxidative stress, lipid peroxidation seriously affects the immune system, making it more sensitive to attack by pathogens (whether viral or produced by *Leishmania major* (leishmaniasis), but which in turn increases the rate of cell death (Matsushita et al., 2015). It has also been seen that the supply of vitamin E, a powerful antioxidant, reduces lipid peroxidation, which in turn reduces the rate of cell death and senescence (Matsushita et al., 2015). Here it has been proven that the use of these lipid extracts with antioxidant activity from Iberian ham fat produced a strong reduction in cell death, with an increase in the growth rate, indicating recovery of redox balance and recovery from cellular damage.

Specifically, the study carried out has shown that with 1 $\mu$l of any of the different extracts analysed there is a notable recovery of biomass produced, growth rate and the number of viable cells (survivors), compared to the control sample subjected to oxidative stress, by 57-63% in the growth rate and by 54-59% in the number of viable cells (survivors). This means that the *in vivo* antioxidant effect is produced with 1 $\mu$l of extract in a final volume of 200 $\mu$l, and the prevention of cellular oxidative stress and improvement of longevity and prevention of cellular aging.

**Example 9: Example of preparation of a pharmaceutical composition object of the present invention from the extracts of Example 8**

Continuing with the indications given throughout this specification, 1 gram of the pharmaceutical composition was prepared, requiring between 750-950 mg of any of the extracts of Example 8, to which the excipients were added using common and known techniques in the field, which were: bulking agents, maltodextrin, tocopherols and purified water. This composition comes in capsule form.

**Claims**

1.  An extract from cured pork ham fat.

2.  The extract according to claim 1, wherein the fat from the pork ham is fat trimmings.

3.  The extract according to any one of claims 1 or 2, wherein the ham is made from Iberian pork.

4.  The extract according to any one of the preceding claims, wherein the pig is acorn-fed, during the montanera period.

5.  The extract according to any one of the preceding claims, having at least one monounsaturated fatty acid in an amount between 60% and 62% by weight with respect to the total weight of the extract.

6.  The extract according to the preceding claim, wherein the at least one monounsaturated fatty acid is oleic fatty acid, and is present in an amount comprised between 55% and 59% by weight with respect to the total weight of the extract.

7.  The extract according to any one of claims 5 or 6, having at least one saturated fatty acid in an amount comprised between 30% and 33% by weight with respect to the total weight of the extract.

8.  The extract according to the preceding claim, wherein the at least one saturated fatty acid is palmitic acid and is present in an amount comprised between 19% and 21% by weight with respect to the total weight of the extract, and a second saturated fatty acid is stearic acid, and is present in an amount comprised between 8% and 10.5% by weight with respect to the total weight of the extract.

9.  The extract according to any one of claims 5 to 8, having at least one polyunsaturated fatty acid in an amount comprised between 7% and 10% by weight with respect to the total weight of the extract.

10. The extract according to the preceding claim, wherein the at least one polyunsaturated fatty acid is linoleic acid and is present in an amount comprised between 6% and 9% by weight with respect to the total weight of the extract, and a second polyunsaturated fatty acid is $\alpha$-linolenic acid and is present in an amount less than 0.5% to 1.5% by weight with respect to the total weight of the extract.

11. The extract according to any one of the preceding claims, which is presented in one of the forms selected within the group made up of: dry, dehydrated, emulsified, diluted, lyophilised, atomised, centrifuged extract and extract in the form of fat.

12. A method for producing the pork ham fat extract described according to any one of the preceding claims, **characterised in that** it comprises:

> - heating the fat under vacuum conditions with rotation, at a pressure comprised between 55 and 930 mbar and at a temperature comprised between 30°C-90°C; with a rotation speed comprised between 80-90 rpm; and for an estimated time comprised between 40 minutes and 130 minutes.

13. The method according to the preceding claim, wherein the pressure is comprised between 90 and 120 mbar, the temperature is comprised between 30°C and 85°C and the time is comprised between 45 minutes and 120 minutes.

14. The method according to the preceding claim, wherein the pressure is 100 mbar and the temperature is between 60 and 62°C.

15. The method according to any one of claims 12 to 14, comprising storing the obtained extract at a temperature comprised between 2°C and -22°C.

16. A food composition comprising the extract described in any one of claims 1 to 11.

17. The food composition according to the preceding claim, comprising the extract in an amount comprised between 1% and 50% by weight of the total weight of the food composition.

18. The food composition according to claim 17, which is the pork ham fat extract.

19. The food composition according to any one of claims 16 to 18, which is selected between a food and a food ingredient.

20. The food composition according to the preceding claim, which is selected between a nutraceutical food and a seasoning and/or flavouring.

21. The food composition according to claim 19, which is a food selected from the group consisting of: meat product, dairy product, dairy derivative, sauce, food paste, baking and pastry product; and any mixture thereof.

22. A use of the pork ham fat extract described according to any one of claims 1 to 11 as a food composition.

23. The use according to the preceding claim, wherein the composition is selected from a food or a food ingredient.

24. The use according to the preceding claim, wherein the food is a nutraceutical food.

25. The use according to claim 23, wherein the food ingredient is a seasoning and/or flavouring.

26. A pharmaceutical composition comprising the extract from cured pork ham fat according to any one of claims 1 to 11 as an active ingredient, in an amount comprised between 75% and 95% by weight of the total weight of the composition.

27. The pharmaceutical composition according to claim 26, comprising the cured pork ham fat extract as an active ingredient in an amount comprised between 92% and 95% by weight of the total weight of the composition.

28. The pharmaceutical composition according to any one of claims 26 or 27, for its use in medicine.

29. The pharmaceutical composition for use according to claim 28, in the prevention of a disease selected from the group consisting of: cancer, diabetes, cardiovascular disease, inflammatory bowel disease, neurodegenerative disease, dermatological disease and a disease related to the weakening of the immune system.

30. The pharmaceutical composition for use according to the preceding claim, wherein the disease is selected between cardiovascular disease or inflammatory bowel disease.

31. The pharmaceutical composition for use according to any one of claims 29 or 30, wherein a daily dose of said pharmaceutical composition is administered that contains the active ingredient in an amount equivalent to 0.5% of the total number of foods with action against oxidative stress that is ingested daily.

Fig. 1

Fig. 2

## INTERNATIONAL SEARCH REPORT

| International application No. |
| --- |
| PCT/ES2022/070146 |

**A. CLASSIFICATION OF SUBJECT MATTER**

See extra sheet

According to International Patent Classification (IPC) or to both national classification and IPC

**B. FIELDS SEARCHED**

Minimum documentation searched (classification system followed by classification symbols)
A23L, A61K

Documentation searched other than minimum documentation to the extent that such documents are included in the fields searched

Electronic data base consulted during the international search (name of data base and, where practicable, search terms used)

EPODOC, INVENES, WPI, FSTA, INTERNET

**C. DOCUMENTS CONSIDERED TO BE RELEVANT**

| Category* | Citation of document, with indication, where appropriate, of the relevant passages | Relevant to claim No. |
| --- | --- | --- |
| X | JP 2009149599 A (NAKAGIMA K.) 09/07/2009, Abstract. | 1-11, 26-31 |
| A | ES 2213488 A1 (UNIVERSIDAD DE CORDOBA) 16/08/2004, column 2, lines 24-27, 38-45; column 3, lines 31-57; column 4 lines 4-12. | 1-25 |
| A | FERNANDEZ J. et al. A diet based on cured acorn-fed ham with oleic acid content promotes anti-inflammatory gut microbiota and prevents ulcerative colitis in an animal model. Lipids In Health And Disease, 2020, pages 1-19 Abstract; table 2; conclusiones | 1-31 |

☒ Further documents are listed in the continuation of Box C.          ☒ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance. | |
| "E" earlier document but published on or after the international filing date | |
| "L" document which may throw doubts on priority claim(s) or which is cited to establish the publication date of another citation or other special reason (as specified) | "X" document of particular relevance; the claimed invention cannot be considered novel or cannot be considered to involve an inventive step when the document is taken alone |
| "O" document referring to an oral disclosure use, exhibition, or other means. | "Y" document of particular relevance; the claimed invention cannot be considered to involve an inventive step when the document is combined with one or more other documents , such combination being obvious to a person skilled in the art |
| "P" document published prior to the international filing date but later than the priority date claimed | |
| | "&" document member of the same patent family |

| Date of the actual completion of the international search | Date of mailing of the international search report |
| --- | --- |
| 24/06/2022 | (28/06/2022) |
| Name and mailing address of the ISA/ OFICINA ESPAÑOLA DE PATENTES Y MARCAS Paseo de la Castellana, 75 - 28071 Madrid (España) Facsimile No.: 91 349 53 04 | Authorized officer J. López Nieto Telephone No. 91 3498426 |

Form PCT/ISA/210 (second sheet) (January 2015)

**INTERNATIONAL SEARCH REPORT**

| International application No. |
| --- |
| PCT/ES2022/070146 |

C (continuation).                         DOCUMENTS CONSIDERED TO BE RELEVANT

| Category * | Citation of documents, with indication, where appropriate, of the relevant passages | Relevant to claim No. |
| --- | --- | --- |
| A | VENTANAS S. et al. Extensive feeding versus oleic and tocopherol enriched mixed diets for production of Iberian dry-cured ham. Effects on chemical composition oxidative status and sensory traits.. Meat Science VENTANAS, S. et al. Meat Science, 2007, Vol. 77, Pages 246-256. | 1-31 |

Form PCT/ISA/210 (continuation of second sheet) (January 2015)

## INTERNATIONAL SEARCH REPORT

International application No.

PCT/ES2022/070146

CLASSIFICATION OF SUBJECT MATTER

*A23L33/00* (2016.01)
*A61K31/20* (2006.01)
*A61K31/201* (2006.01)
*A61K31/202* (2006.01)

Form PCT/ISA/210 (extra sheet) (January 2015)

## INTERNATIONAL SEARCH REPORT

Information on patent family members

International application No.

PCT/ES2022/070146

| Patent document cited in the search report | Publication date | Patent family member(s) | Publication date |
|---|---|---|---|
| JP2009149599 A | 09.07.2009 | JP4512881B B2 | 28.07.2010 |
| ES2213488 A1 | 16.08.2004 | WO2004071219 A1 | 26.08.2004 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- ES 202131111 P

### Non-patent literature cited in the description

- **VENTANAS, S. ; VENTANAS, J. ; RUIZ, J. ; ESTÉVEZ, M.** Iberian pigs for the development of high-quality cured products. *Res. Devel. Agricultural & Food Chem.,* 2005, vol. 6, 1-27
- **JIMÉNEZ-COLMENERO, F. ; VENTANAS, J. ; TOLDRÁ, F.** Nutritional composition of dry-cured ham and its role in a healthy diet. *Meat Science,* 2010, vol. 84, 585-593
- **RODRÍGUEZ-GONZÁLEZ, M. ; TÁRRAGA, M.L. ; MADRONA, F ; SADEK, I.M. ; CELADA, C. ; TÁRRAGA, P.J.** Effects of the Mediterranean diet on the cardiovascular risk factors. *JONNPR.,* 2019, vol. 4 (1), 25-51
- **MAYORAL, P. ; MARTINEZ-SALGADO, C.S. ; SANTIAGO, J.M. ; RODRÍGUEZ-HERNÁNDEZ, M.V. ; GARCÍA-GÓMEZ, M.L. ; MORALES, A. ; LÓPEZ-NOVOA, J.M. ; MARCÍAS-NÚÑEZ, J.F.** Effect of ham protein substitution on oxidative stress in older adults. *Nutr Health Aging,* 2003, vol. 7 (2), 84-9
- **VENTANAS, S. ; VENTANAS, J. ; TOVAR, J. ; GARCIA, C. ; ESTÉVEZ, M.** Extensive feeding versus oleic acid and tocopherol enriched mixed diets for the production of Iberian dry-cured hams: Effect on chemical composition, oxidative status and sensory traits. *Meat Science,* 2007, vol. 77, 246-256
- **SABAN-RUIZ, J. ; FABREGATE-FUENTE, M. ; FABREGATE-FUENTE, R. ; ANDRÉS-CASTILLO, A. ; PALOMINO, A. ; BARRIO-CARRERAS, D. ; MARTÍN-FERNÁNDEZ, L. ; ALRAMIRANO, F. ; FÉRNANDEZ-FÉRNANDEZ, C. ; ANDRÉS-LACUEVA, C.** Iberian cured-ham consumption improves endothelial function in healthy subjects. *J Nutr Health Aging,* 2017, vol. 21, 1277-1283
- **MARTORELL, P. ; FORMENT, J.V. ; DE LLANOS, R. ; MONTÓN, F. ; LLOPIS, S. ; GONZÁLEZ, N. ; GENOVÉS, S. ; CIENFUEGOS, E. ; MONZÓ, H. ; RAMÓN, D.** Use of Saccharomyces cerevisiae and Caenorhabditis elegans as Model Organisms to Study the Effect of Cocoa Polyphenols in the Resistance to Oxidative Stress. *J. Agric. Food Chem.,* 2011, vol. 59 (5), 2077-2085

- **CAROCHO, M. ; FERREIRA, I.C.F.R.** A review on antioxidants, prooxidants and related controversy: Natural and synthetic compounds, screening and analysis methodologies and future perspectives. *Food and Chemical Toxicology,* 2013, vol. 51, 5-25
- **CAROCHO, M ; FERREIRA, I.C.F.R.** A review on antioxidants, prooxidants and related controversy: Natural and synthetic compounds, screening and analysis methodologies and future perspectives. *Food and Chemical Toxicology,* 2013, vol. 51, 5-25
- **GARCÍA-SÁNCHEZ, A ; MIRANDA-DÍAZ, A.G. ; CARDONA-MUÑOZ, E.G.** The Role of Oxidative Stress in Physiopathology and Pharmacological Treatment with Pro- and Antioxidant Properties in Chronic Diseases. *Hindawi,* 2020, vol. 1-16
- **MATSUSHITA, M. ; FREIGANG, S. SCHNEIDER, C. ; CONRAD, M. ; BORNKAMM, G.W. ; KOPF, M.** T cell lipid peroxidation induces ferroptosis and prevents immunity to infection. *J. Exp. Med.,* 2015, vol. 212 (4), 555-568
- **HUANG, M.Z. ; D LI, J.Y.** Physiological regulation of reactive oxygen species in organisms based on their physicochemical properties. *Acta Physiologica,* 2020, vol. 228, 1-16
- **AYALA, A. ; MUÑOZ, M.F ; ARGÜELLES, S.** Lipid Peroxidation: Production, Metabolism, and Signaling Mechanisms of Malondialdehyde and 4-Hydroxy-2-Nonenal. *Hindaw,* 2014, 1-31
- **MIGUEL, M. ; VASSALLO, D. V. ; WIGGERS, G. A.** Bioactive peptides and hydrolysates from egg proteins as a new tool for protection against cardiovascular problems. *Current Pharmaceutical Design,* 2020, vol. 26 (30), 3676-3683
- **MAYORAL, P. ; MARTINEZ-SALGADO, C.S. ; SANTIAGO, J.M. ; RODRÍGUEZ-HERNÁNDEZ, M.V. ; GARCIA-GÓMEZ, M.L. ; MORALES, A. ; LÓPEZ-NOVOA, J.M. ; MARCÍAS-NUÑEZ, J.F.** Effect of ham protein substitution on oxidative stress in older adults. *Nutr Health Aging,* 2003, vol. 7 (2), 84-9

- **HUANG, D. ; OU, B. ; PRIOR, R.L.** The Chemistry behind Antioxidant Capacity Assays. *J. Agric. Food Chem.,* 2005, vol. 53, 1841-1856
- **MACDONALD-WICKS, L.K. ; WOOD, L.G. ; GARG, M.L.** Methodology for the determination of biological antioxidant capacity in vitro: a review. *J Sci Food Agric,* 2006, vol. 86, 2046-2056
- **MORALES, D. ; MIGUEL, M. ; GARCÉS-RIMÓN, M.** Pseudocereals: a novel source of biologically active peptides. *Critical Reviews in food science and nutrition,* 2021, vol. 61 (9), 1537-1544
- **VETTORAZZI, A ; LÓPEZ DE CERAIN, A. ; SANZ-SERRANO, J. ; GIL, A.G ; AZQUETA, A.** European Regulatory Framework and Safety Assessment of Food-Related Bioactive Compounds. *Nutrients,* 2020, vol. 12 (613), 1-16
- **SNEDDON, L.U. ; HALSEY, L.G. ; BURY, N.R.** Considering aspects of the 3Rs principles within experimental animal biology. *Journal of Experimental Biology,* 2017, vol. 220, 3007-3016
- **SMITH, M.G. ; SNYDER, M.** Yeast as a Model for Human Disease. *Current Protocols in Human Genetics,* 2006, vol. 48, 15.6.1-15.6.8
- **BOLOTIN-FUKUHARA, M. ; DUMAS, B. ; GAILLARDIN, C.** Yeasts as a model for human diseases. *FEMS Yeast Res.,* 2010, vol. 10, 959-960
- **KARATHIA, H. ; VILAPRINYO, E. ; SORRIBAS, A. ; ALVES, R.** Saccharomyces cerevisiae as a model organism: a comparative study. *PloS one,* 2011, vol. 6 (2), 1-10
- **PÉREZ DE VEGA, M.J. ; MORENO-FERNÁNDEZ, S. ; PONTES-QUERO, G.M ; GONZÁLEZ-AMOR, M. ; VÁZQUEZ-LASA, B. ; SABATER-MUÑOZ, B. ; BRIONES, A.M. ; AGUILAR, M.R. ; MIGUEL, M. ; GONZÁLEZ-MUÑIZ, R.** Characterization of Novel Synthetic Polyphenols: Validation of Antioxidant and Vasculoprotective Activities. *Antioxidants,* 2020, vol. 9 (787), 1-23
- Stress Biology of Yeasts and Fungi. 2015
- **ELEUTHERIO, E. ; DE ARAUJO BRASIL, A. ; BRAGA FRANÇA, M. ; SEIXAS GOMES DE ALMEIDA, D. ; BREVES RONA, G. ; SILVA MAGALHAESS, R.S.** Oxidative stress and aging: Learning from yeast lessons. *Fungal Biology,* 2018, vol. 122, 514-525
- **BAYLIAK, M.M. ; BURDYLYUK, N.I. ; LUSHCHAK, V.I.** Quercetin increases stress resistance in the yeast Saccharomyces cerevisiae not only as an antioxidant. *Ann Microbiol.,* 2016, vol. 66, 569-576
- **MENG, D. ; ZHANG, P. ; LI, S. ; HO, C.T. ; ZHAO, H.** Antioxidant activity evaluation of dietary phytochemicals using Saccharomyces cerevisiae as a model. *Journal of Functional Foods,* 2017, vol. 38, 36-44
- **GAO, Y. ; FANG, L. ; WANG, X. ; LAN, R. ; WANG, M. ; DU, G. ; GUAN, W. ; LIU, J. ; BRENNAN, M. ; GUO, H.** Antioxidant Activity Evaluation of Dietary Flavonoid Hyperoside Using Saccharomyces Cerevisiae as a Model. *Molecules,* 2019, vol. 24 (788), 1-12
- **GARC6S-RIM6N, M. ; GONZÁLEZ, C. ; URANGA J.A. ; LÓPEZ-MIRANDA, V. ; LÓPEZ-FANDIÑO, R. ; MIGUEL, M.** Pepsin Egg White Hydrolysate Ameliorates Obesity-Related Oxidative Stress, Inflammation and Steatosis in Zucker Fatty Rats. *PLoS ONE,* 2016, vol. 11 (3), 1-15
- **NINFALI, P. ; ALUIGI, G. ; BACCHIOCCA, M. ; MAGNANI, M.** Antioxidant Capacity of Extra-Virgin Olive Oils. *JAOCS,* 2001, vol. 78 (3), 243-247
- **FERNÁNDEZ, J. ; GARCIA DE LA FUENTE, V. ; FERNÁNDEZ GARCIA, M.T. ; GÓMEZ SÁNCHEZ, J. ; ISABEL REDONDO, B. ; VILLAR, C.J. ; LOMB, F.** A diet based on cured acorn-fed ham with oleic acid content promotes anti- inflammatory gut microbiota and prevents ulcerative colitis in an animal model. *Lipids in Health and Disease,* 2020, 1-19
- **MAYORAL, P. ; MARTINEZ-SALGADO, C. ; SANTIAGO, J. ; RODRIGUEZ-HERNANDEZ, M. ; GARCÍA-GÓMEZ, M. ; MORALES, A. ; LÓPEZ-NOVOA, J. ; MACÍAS-NÚÑEZ, J.** Effect of Ham Protein Substitution on Oxidative Stress in Older Adults. *J. Nutr. Health & aging,* 2003, vol. 7 (2), 84-89
- **MAYORAL, P. ; MARTINEZ-SALGADO, C ; SANTIAGO, J. ; RODRÍGUEZ-HERNÁNDEZ, M. ; GARCÍA-GÓMEZ, M. ; MORALES, A. ; LÓPEZ-NOVOA, J. ; MACÍAS-NÚÑEZ, J.** Effect of Ham Protein Substitution on Oxidative Stress in Older Adults. *J. Nutr. Health & aging,* 2003, vol. 7 (2), 84-89
- **FERNÁNDEZ, J. ; GARCIA DE LA FUENTE, V. ; FERNÁNDEZ GARCIA, M. T. ; GÓMEZ SÁNCHEZ, J. ; ISABEL REDONDO, B ; VILLAR, C.J. ; LOMBÓ, F.** A diet based on cured acorn-fed ham with oleic acid content promotes anti- inflammatory gut microbiota and prevents ulcerative colitis in an animal model. *Lipids in Health and Disease,* 2020, vol. 19 (28), 1-19
- **ISABEL, B. ; CORDERO, G. ; OLIVARES, A. ; DAZA, A. ; LOPEZ-BOTE, C.J.** Differential response of Iberian and lean pig crossbreeds to dietary linoleic acid administration. *Spanish Journal of Agricultural Research,* 2014, vol. 12 (2), 419-426
- **ARIZA, J.A. ; LÓPEZ., F. ; COYOTL., J. ; RAMOS, ME. ; DIAZ, J. ; MARTINEZ, A.** Effect of different extraction methods on the fatty acid profile in the avocado (Persea americana Mill. var. Hass) oil. *Venezuelan Magazine of Food Science and Technology,* 2011, vol. 2 (2), 263-276

- **NGAMPEERAPONG, C ; CHAVASIT, V. ; DURST, R.W.** Bioactive and nutritional compounds in virgin coconut oils. *Mal J Nutr,* 2018, vol. 24 (2), 257-267
- **GIUFFRÈ, A.M. ; CARACCIOLO, M. ; ZAPPIA, C. ; CAPOCASALE, M. ; POIANA, M.** Effect of heating on chemical parameters of extra virgin olive oil, pomace olive oil, soybean oil and palm oil. *Ital. J. Food Sci.,* 2018, vol. 30, 715-739

- **THIMOTEO, D ; BRAZ, R. ; RIBEIRO, R. ; DE LACERDA, L. ; STEDEFELDT, E.** Methods for applying the tests of acceptability for school feeding: the validation of playful cards. *Rev Chil Nutr,* 2013, vol. 40 (4), 357-363
- **SPROUFFSKE ; WAGNER.** *RStudio 2021,* 2016
- **SPROUFFSKE, K. ; WAGNER, A.** Growthcurver: an R package for obtaining interpretable metrics from microbial growth curves. *BMC Bioinformatics,* 2016, vol. 17 (172), 2-4